Europäisches Patentamt

European Patent Office

Office européen des brevets

(19)

(11) Veröffentlichungsnummer: **0 035 586**
**B1**

(12)  # EUROPÄISCHE PATENTSCHRIFT

(45) Veröffentlichungstag der Patentschrift: **13.06.84**

(21) Anmeldenummer: **80101275.8**

(22) Anmeldetag: **12.03.80**

(51) Int. Cl.³: **A 61 K 35/02,**
**A 61 K 31/28**

(54) Neuer biochemischer Wirkstoff, Verfahren zu seiner Herstellung und diesen Wirkstoff enthaltende pharmazeutische Zubereitung.

(43) Veröffentlichungstag der Anmeldung:
**16.09.81 Patentblatt 81/37**

(45) Bekanntmachung des Hinweises auf die Patenterteilung:
**13.06.84 Patentblatt 84/24**

(84) Benannte Vertragsstaaten:
**AT BE CH DE FR GB IT LU NL SE**

(56) Entgegenhaltungen:
US - A - 4 151 207

CHEMISCHE BERICHTE, Band 89, Nr. 9, 1956, Seiten 2168-2173 Weinheim, DE. K. FREUDENBERG et al.: "Anwendung radioaktiver Isotope bei der Erforschung des Lignins, VI. Herkunft der Isohemipinsäure"
CHEMISCHE BERICHTE, Band 95, 1962, Seiten 2814-2828 Weinheim, DE. K. FREUDENBERG et al.: "Die Oxydation des methylierten natürlichen und künstlichen Lignins"
CHEMICAL ABSTRACTS, Band 82, Nr. 22, 2. Juni 1975, Seite 102, Nr. 141821y Columbus, Ohio, U.S.A. N.A. MEKLER et al.: "Oxidative degradation of lignin in an alkaline medium"

(73) Patentinhaber: **Mach, Walter, Dr., Dipl.-Phys.**
**Wasserburger Strasse 17**
**D-8011 Kirchseeon (DE)**

(72) Erfinder: **Mach, Walter, Dr., Dipl.-Phys.**
**Wasserburger Strasse 17**
**D-8011 Kirchseeon (DE)**

(74) Vertreter: **Berg, Wilhelm, Dr. et al,**
**Dr. Berg, Dipl.-Ing. Stapf, Dipl.-Ing. Schwabe, Dr. Dr. Sandmair Mauerkircherstrasse 45**
**D-8000 München 80 (DE)**

(56) Entgegenhaltungen:
CHEMICAL ABSTRACTS, Band 88, Nr. 14, 3 April 1978, Seite 302, Nr. 94369u Columbus, Ohio, U.S.A. M. BODZEK et al.: "Application of reverse osmosis and ultrafiltration for pulp and paper industry effluent treatment. III. Separation and concentration of lignin compound solutions by ultrafiltration"
CHEMICAL ABSTRACTS, Band 90, Nr. 24, 11. Juni 1979, Seite 356, Nr. 192428y Columbus, Ohio, U.S.A. V.I. SHARKOV et al.: "Use of hydrolyzed lignin in medicine"
ROTE LISTE, 1963, Editio Cantor, Alendorf/Württ.

Courier Press, Leamington Spa, England.

**Beschreibung**

Die Fähigkeit des lebenden Organismus, exogene Belastungen erfolgreich zu überwinden, hängt von der Güte aller biochemischen Systeme und deren konzentriertem Zusammenwirken ab. Dieses Zusammenwirken ist bekanntlich eine von der Parität kataboler und anaboler Regulationsfaktoren abhängige Leistungsgröße, die z.B. durch verschiedene exogene Belastungsfaktoren wie z.B. psychosozialer Streß, Bakterien, Viren usw. vermindert wird. Auch ist bei schwacher zellulärer Leistungsfähigkeit die Wirkung hochspezifischer Arzneimittel wie z.B. von Antibiotika gering und die tragenden reparativen Prozesse wie Infektionsabwehr, gewebliche Regeneration und Entzündungsausheilung nehmen eine unzureichenden Ablauf.

Um die allgemeine Belastungsüberwindungsfähigkeit zu erhöhen, hat die medizinische Wissenschaft bisher nur wenige und verhältnismäßig ungezielte Methoden zur Verfügung, wie z.B. die Gabe von Vitaminen, unspezifischen Reizkörpern und bestimmten Gewebs- oder Blutextrakten. In gewissen Fällen werden auch körperliche Trainingskuren eingesetzt. Es ist jedoch bekannt, daß beispielsweise unspezifische Reizkörper und ein Muskeltraining zuerst einmal eine im einzelnen unbekannte Reaktionskette in Ganz setzen, an deren Ende erst die erhoffte Wirkung stehen kann. Die bekannten unspezifischen Reizkörper bedeuten für den meist ohnehin schon geschwächten Organismus eine zusätzliche Belastung, die sich im Anstieg der Körpertemperatur, erhöhtem Krankheitsgefühl, negativen Auswirkungen auf bestimmte Grund- oder Begleitkrankheiten, wie Allergien, Schliddrüsenfunktionsstörungen, Diabetes usw. auswirkt.

Es besteht deshalb ein Bedürfnis nach einer Wirksubstanz, die unmittelbar an einem Grundprozeß der intrazellulären Stoffwechselregulation eingreift, so daß die Belastungsüberwindungsfähigkeit ohne Belastungsphase und ohne toxisches Risiko rasch ansteigt. Eine solche Wirkung sollte nicht auf bestimmte Gewebe-Typen beschränkt, sondern gewebeunspezifisch sein.

Es wurde nunmehr gefunden, daß diese biologischen Wirkungen in therapeutisch nutzbarem Ausmaß durch einen neuen biochemischen Wirkstoff erzielt werden könne, der sich von nativen und/oder synthetischen lignoiden Dehydrierungspolymerisaten (DHPs) ableiten läßt und der die kennzeichnenden chemischen Strukturen des Lignins in alkali- und wasserlöslicher Form enthält.

Lignine sind schwer lösliche Dehydrierungspolymerisate (DHPs) aus Monolignolen, chemisch höchst inert und biologisch inaktiv. Die erfindungsgemäße Substanz wird dadurch erhalten, daß man natives oder synthetisches Lignin zusammen mit Kohlehydraten — vorzugsweise Mono- und/oder Oligosaccharide, insbesondere Glucose — in alkalisch-wässrigem Milieu in Gegenwart von wasserunlöslichen, anorganischen Oxidationskatalysatoren mit Ozon, Sauerstoff und/oder Wasserstoffperoxid — vorzugsweise Luft — oxidiert, durch Druckfiltration an Membranen mit nominellen Trennschnittgrenzen von 5000 bis 35000, vorzugsweise 10000 bis 25000 Dalton fraktioniert und anschließend durch weitere Reinigungsschritte gewinnt.

Synthetische Lignine* wurden bisher lediglich zum Zweck der Konstitutionsaufklärung durch enzymatische Dehydrierung von Monolignolen dargestellt. Je nach Wahl der Ausgangsprodukte (Monolignole: p-Cumar-, Coniferyl- und Sinapinalkohol) und ihrem Verhältnis zueinander lassen sich lignoide DHPs vom Laubholz-, Nadelholz- oder Graslignin-Typ darstellen. Auch durch die Methodik der DHP-Synthese lassen sich unterschiedliche Typen darstellen, je nachdem man das Zutropf- oder das Zulaufverfahren oder eine Kombination beider Verfahren zur Anwendung bringt. Als Reduktase findet meist Laccase aus dem Saft des Pilzes Psalliota campestria Verwendung.

Als Lignine können erfindungsgemäß Syntheseprodukte aus Monolignolen wie p-Cumar-, Coniferyl- und/oder Sinapinalkohol nach dem Zutropf-, dem Zulauf- oder dem Kombinationsverfahren sowie native Lignine wie Björkmann-Lignin, Brauns-Lignin, Wildstätter-Lignin — vorzugsweise aber Nord's-Lignin — verwendet werden. Mit Nord's-Lignin werden die höchsten Ausbeuten erhalten. Nicht geeignet sind andere lösliche Lignin-Derivative wie Säure-Lignin, Cuproxan-Lignin bzw. Lignin-Sulfonsäure, da sie offenbar nicht die erforderlichen Strukturelemente enthalten.

Als Kohlehydrate werden Mono- und Oligosaccharide, insbesondere Mono- und Disccharide — vorzugsweise Glucose — eingesetzt.

Als Oxidationskatalysatoren sind anorganische Oxidationskatalysatoren geeignet, die eine hohe Katalaseaktivität aufweisen, wie z.B. Mangandioxide. Da die erfindungsgemäße Mischrepolymerisation zu Mischrekombinaten nur dann mit wirtschaftlich tragbarem Erfolg abläuft, werden erfindungsgemäß wasserunlösliche, anorganische Oxidationskatalysatoren eingesetzt, die sich ohne Schwierigkeit durch herkömmliche Methoden der Feststofftrennung von den alkalilöslichen, organischen Mischrekombinaten abtrennen lassen. Bei dieser oxidativen Radikalpolymerisation zu lignoiden Reduktion-Mischrekombinaten, bilden sich naturgemäß Systeme sehr unterschiedlichen Molekulargewichts, die sich zum Teil durch intermolekulare Wechselbeziehung gegenseitig inhibieren, was ihre biologische Aktivität betrifft. Es wird deshalb erfindungsgemäß durch eine aktive Molekulartrennung nach dem Prinzip der Ultrafiltration unter Druck eine Isolierung niedermolekularer, biochemisch hoch reaktiver Rekombinate vorgenommen und das hierbei anfallende Retinat verworfen.

---

*Freudenberg, Holzfurschung *18*, H6, S. 167 (1964)

**0 035 586**

Nachfolgend wird anhand des beigefügten Fließschemas das erfindungsgemäße Verfahren allgemein erläutert:

Die vorgenannten synthetischen oder nativen Lignine werden zusammen mit einem geeigneten Kohlehydrat — vorzugsweise Glucose — und einem geeigneten anorganischen Oxidationskatalysator mit hoher Katalaseaktivität gemischt und mit verdünnter Natronlauge angeteigt. Man setzt verdünnte Ammoniaklösung zu, bis eine rührfähige Dispersion entsteht. Bei Raumtemperatur wird unter Rühren das Oxidationsmittel — vorzugsweise Luft durch eine Tauchfritte — zugeführt. Die Reaktion ist beendet, wenn der pH-Wert um 1 bis 2 Einheiten abgesunken ist. Dies ist erfahrungsgemäß nach 15 bis 18 Monaten der Fall. Die ungelösten Bestandteile werden sedimentiert und verworfen. Die flüssige Phase wird bis zum Trocknen eingeengt (vgl. Fließschema (1) und (2)).

Das getrocknete Zwischenprodukt wird mit 10 bis 20 Teilen 2 %iger bis 1 %iger Natronlauge suspendiert und teilweise aufgelöst. Der ungelöste Anteil wird nach den üblichen Methoden abgetrennt und verworfen. Mit einem Kationenaustauscher wird in der wässrigen Phase ein pH-Wert von 9,5 bis 14 — vorzugsweise 10,5 bis 11,5 — eingestellt (vgl. Fließschema (3), (4) und (5)).

Alle Substanzen bis zu einem Molekulargewicht von 5000 werden durch Molekulartrennung an geeigneten Membranen, vorzugsweise an Membranen mit nominellen Trennschnittgrenzen von 5000 bis 35000, vorzugsweise 10000 bis 25000 Daltons isoliert (vgl. Fließschema (6)).

Das so erhaltene Filtrat wird entweder durch Zugabe eines geeigneten Kationenaustauschers in der $H\pm$Form auf einen pH von 4,5 bis 6,0 gebracht und ist als solches verwendbar oder kann vorzugsweise durch Zugabe eines geeigneten Kationenaustauschers in der $H\pm$Form auf einen pH-Wert von 2,1 gebracht werden. Der Ionenaustauscher wird dann abgetrennt und die Lösung zentrifugiert (vgl. Fließschema (7)). Das gelartige Sediment und die überstehende Lösung werden getrennt weiterverarbeitet.

a) Das Sediment wird in verdünnter Alkalilauge bei pH 9 gelöst. Die klare alkalische Lösung wird durch Zugabe eines geeigneten Kationenaustauschers auf pH 5,5 zurückgestellt (vgl. Gließschema (8a)).

b) Die überstehende Lösung wird durch Erwärmen auf 95°C und rasches Abkühlen auf 5°C einem Thermoschock ausgesetzt. Man trennt vom entstehenden Sediment und bringt die überstehende Lösung durch Zugabe von Alkalilauge auf pH 5,5 (vgl. Fließschema (8b)).

Die nach a) und b) erhaltenen Lösungen vom pH 5,5 werden gemischt und unter schonenden Bedingungen zur Trockne eingeengt. Der verbleibende Rückstand stellt die erfindungsgemäße Wirksubstanz dar (vgl. Fließschema (9)).

Die erfindungsgemäße Wirksubstanz ist eine nichtkristallisierbare, röntgenamorphe, wasserlösliche organische Säure. Die Substanz ist hell- bis dunkelbraun gefärbt und zeigt einen hohen metallischen Glanz, der auf ungepaarte Elektronen hinweist. Es handelt sich um ein Polymerengemisch, das durch Rekombination der oxidativen Ligninabbauprodukte unter Einbau der Zuckerreduktone gebildet wird. Die Polymeren enthalten redoxamphotere Strukturen mit ungepaarten Elektronen (ESR-Spektrum) sowie phenolische OH-Gruppen, Carboxylfunktionen, Ketogruppen und Endiolgruppierungen, die auch in wässriger Lösung erhalten bleiben. Als typisches Ligninderivat kann der erfindungsgemäße Wirkstoff ebenso wie die Lignine nicht durch eine Strukturformel exakt beschrieben werden. Auch die meisten konventionellen Analysendaten wie Schmelzpunkt, Kristallstruktur, Infrarotspektrum und Summenformel können bei dieser Substanz nicht zur Charakterisierung herangezogen werden. Dagegen kann der neue Wirkstoff durch folgende Methoden charakterisiert werden:

1. Nachweis auch in wässriger Lösung stabiler ungepaarter Elektronen durch ESR-Spektroskopie (ESR-Spektren).
2. Das Fluoreszenz-Spektrum (Emission) in wässriger Lösung.
3. Den Quotienten der Extinktionen bei 375 und 450 nm in wässriger Lösung bei pH 5,5, der von der Feinstruktur der Membran und ihrer nominellen Trennschnittgrenze abhängig ist.
4. Spezifische Vermehrung lichtabsorbierender Gruppen durch Alkalisierung einer wässrig-sauren Lösung.
5. Das Molekulargewichtsverteilungsprofil: 500—5000 (Mittelwert 2000).
6. Das Aussehen des Trockenstoffes ist dunkelbraun.
7. Beeinflussung des Glutathionredoxstatus der lebenden Leberzelle im biochemischen Test.
8. Der Schwimm-Leistungstest.

Erläuterungen zu:

1.) ESR-Spektren

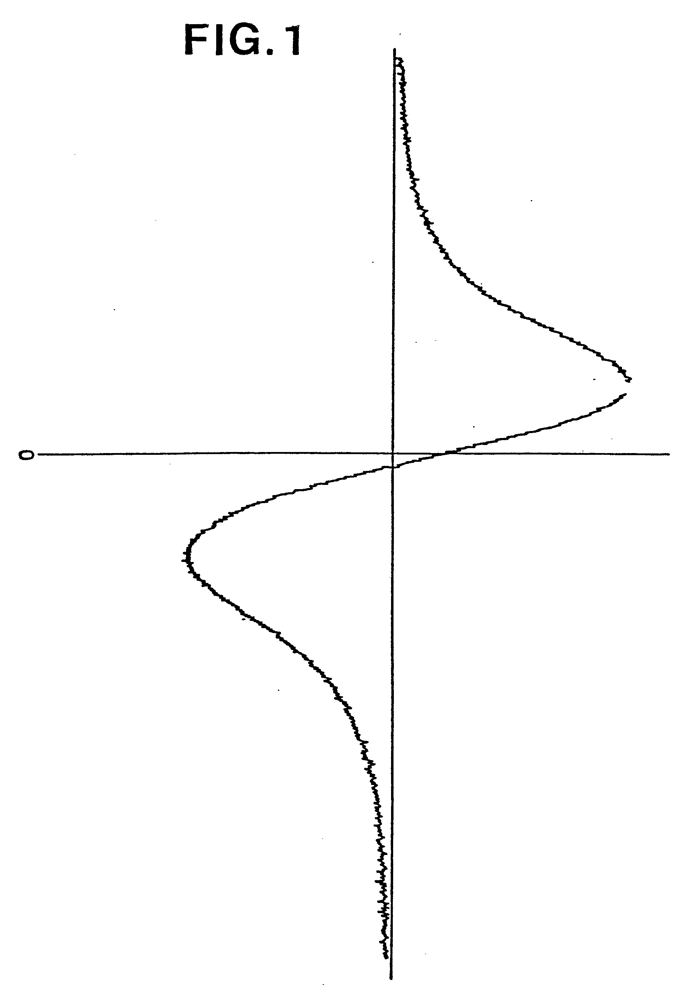

Der Festkörper zeigt ein intensives ESR-Signal (vgl. Abb. 1), das aus einer Linie mit der peak-topeak-Breite von 3,2 Gauß besteht. Der g-Faktor wurde zu 2,0042 bestimmt.

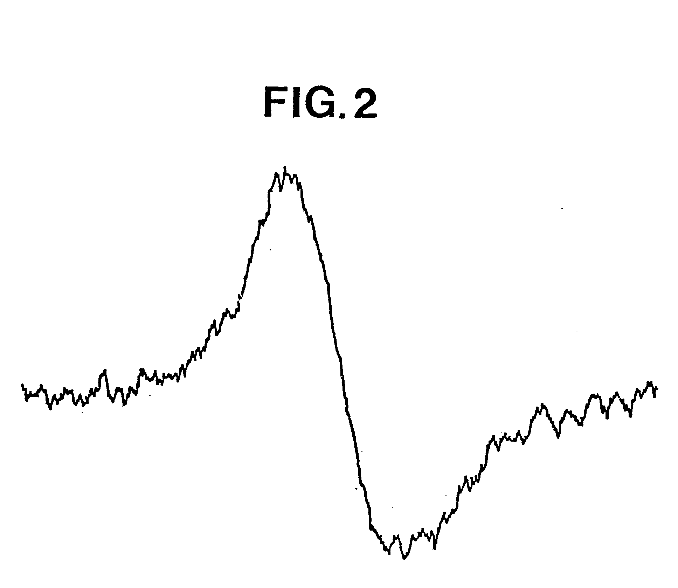

Die Untersuchungen der wässrigen Lösungen wurden bei einem pH-Wert von 9 durchgeführt. Die 5 %igen Lösungen zeigen ein kräftiges Ein-Linien-Signal, das leicht unsymmetrisch ist (vgl. Abb. 2). Die Linienbreite wurde zu 4,2 Gauß ermittelt. Der Nulldurchgang des Signals entspricht einem g-Faktor von

3

2,0045 und ist somit dem Festkörper recht ähnlich. Die Fehlergrenze wird durch die Linienbreite bestimmt. Die Signalintensität zeigt eine nicht-lineare Abhängigkeit von der Konzentration. Des weiteren ist eine Steigerung der experimentell ermittelten Signalintensitäten sowohl durch Einwirkung von Luftsauerstoff als auch nach Zugabe von Ascorbinsäure klar zu erkennen. Bei Zusatz von 1 ml 10 %iger Ascorbinsäure-Lösung zu 3 ml 5 %iger Substanz-Lösung wird eine Steigerung der Signalintensität um den Faktor 2 beobachtet. Die Linienbreite wird durch den Zusatz von Ascorbinsäure praktisch nicht verändert, während der g-Faktor auf 2,0037 absinkt. Gleichzeitig ist eine weitgehende Symmetrierung des in Form der ersten Ableitung registrierten Absorptionssignals zu beobachten.

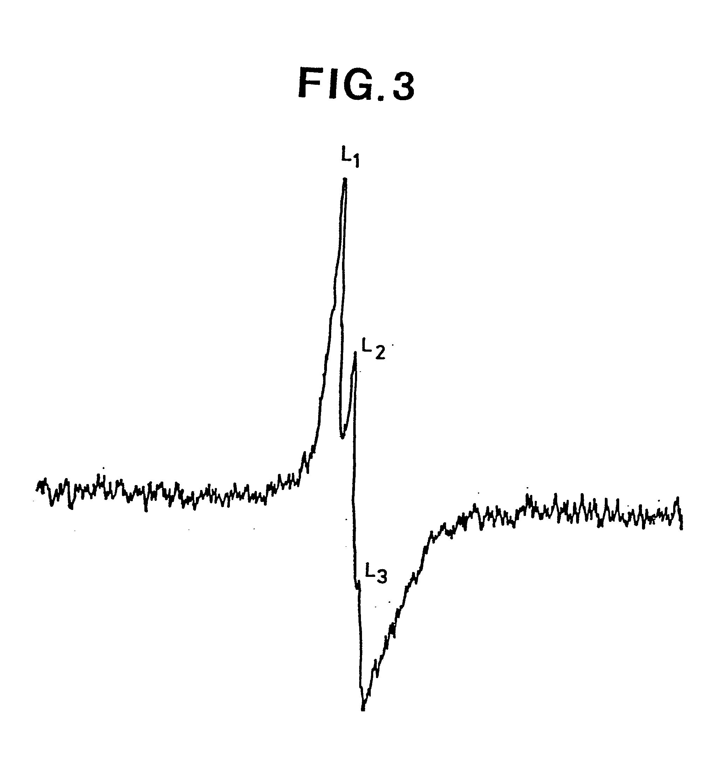

Die Absorption der wässrigen Lösung ohne Pufferzusatz liegt bei nahezu gleichem g-Faktor, wie er beim Festkörper gefunden wurde, ist aber mit einer Linienbreite nach 3,8 G schmaler. Die Linienform ändert sich nach Spülen der Meßprobe mit Stickstoff und nach Optimierung der Feldmodulation ist eine Andeutung einer Aufspaltung erkennbar. Der Zusatz von Kaliumchlorid ist ohne Einfluß auf das Signal. Erhöhung des pH-Wertes auf ca. 10 führt zu drastischen Veränderungen (Abb. 3). Jetzt lassen sich drei scharfe Linien $L_1$, $L_2$, $L_3$ erkennen mit den entsprechenden g-Faktoren $g_1=2,0051$, $g_2=2,0048$ bzw. $g_3=2,0045$. Die Breiten dieser drei Komponenten sind vergleichbar und liegen bei 150 mG. Bei sorgfältiger Untersuchung des Signals zeigt sich, daß unter diesen drei Linien eine vierte, wesentlich breitere ($\Delta H$ 1, 5G) Absorption vorhanden ist. Nach Luftoxidation nimmt die Intensität ab, um nach Zusatz von Glucose auf den alten Wert anzusteigen. Mit fortschreitender Reduktionszeit ändern sich die relativen Intensitäten aller vier Komponenten. Offenbar handelt es sich bei der erfindungsgemäßen Wirksubstanz um ein redoxamphoteres System, das stark pH-abhängig ist. Für die ESR-Spektren sind vermutlich mehrere Radikale verantwortlich, die sich in Redox- und Protonierungsgleichgewichten befinden.

2.) Fluoreszenzspektren

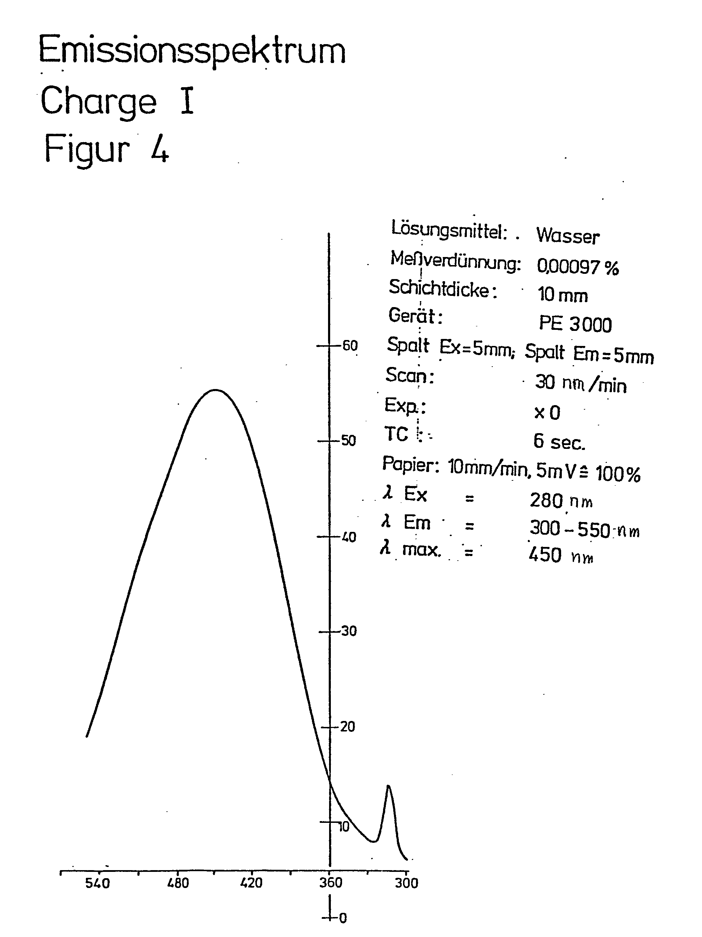

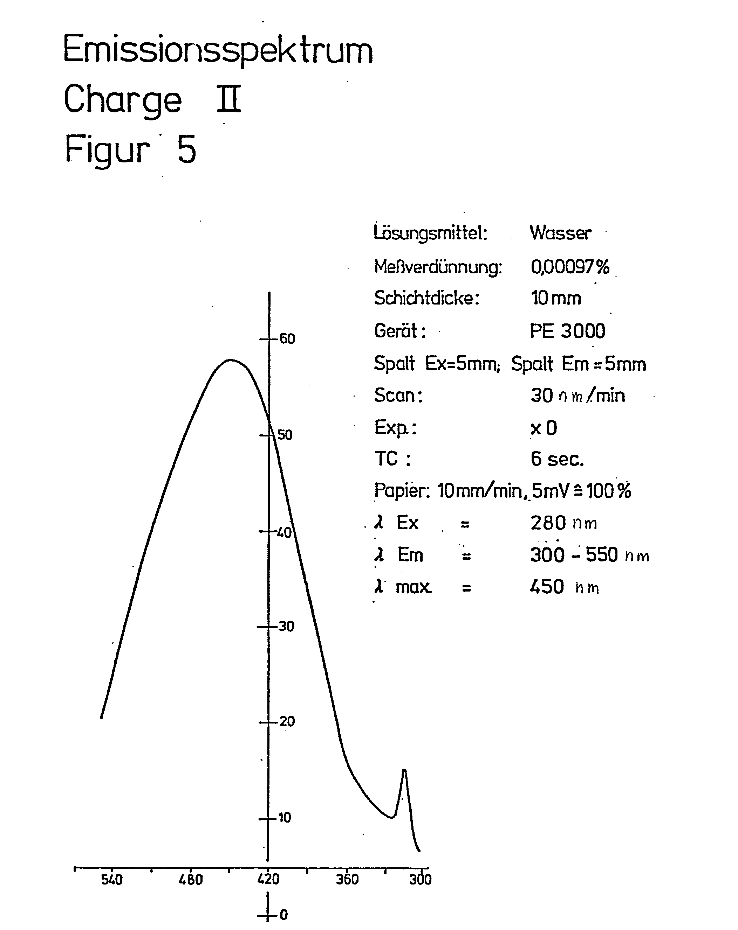

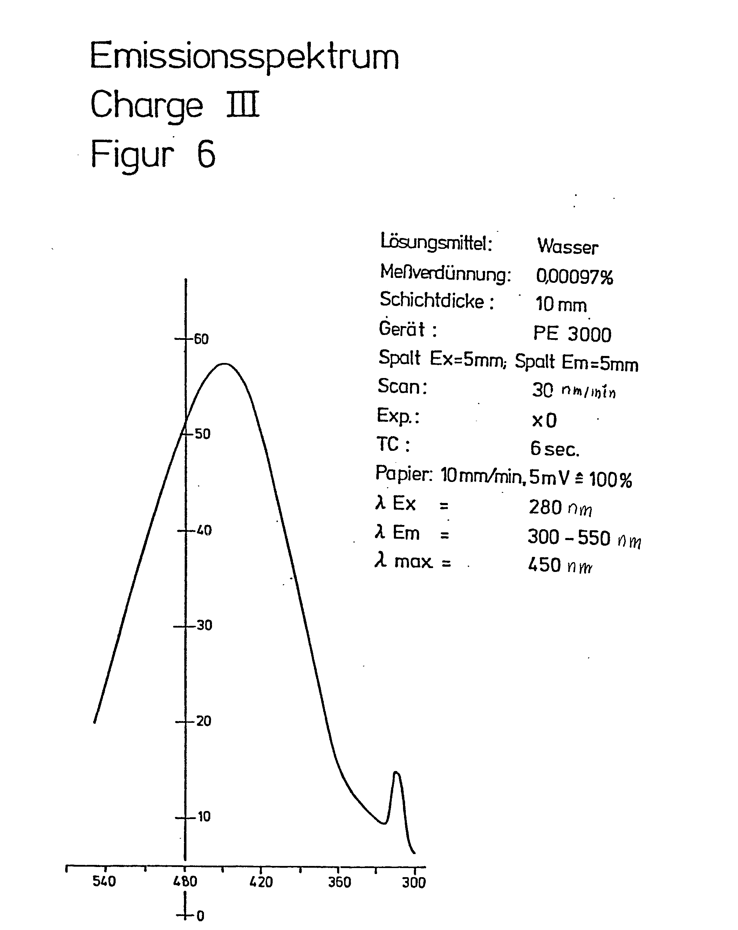

Wenn auch Fluoreszenzspektren wenig über die Struktur einer Verbindung aussagen, so dienen sie doch zur Charakterisierung. Die Emissions spektren der erfindungsgemäßen Wirksubstanz wurden in wässriger Lösung mit dem Spektralfluorimeter PE 3000 der Firma PERKIN ELMER aufgenommen. Insgesamt drei Proben aus verschiedenen Produktionsansätzen ergaben identische Emissions- und Anregungsspektren. Die Spektren sind in den Abbildungen 4 bis 6 wiedergegeben. Die Wirksubstanz weist ein Emissions-Maximum bei 450±4 nm auf.

3.) Quotient der Extinktionen $E_{375nm}$ und $E_{450nm}$ wässriger Lösungen der Wirksubstanz bei pH 5,5

Zur weiteren Charakterisierung der erfindungsgemäßen Wirksubstanz können die Extinktionen wässrig-saurer Lösungen herangezogen werden. Der Quotient der Extinktionen bei $\lambda=375$ nm und $\lambda=450$ nm nimmt dabei bei gleichen Membraneigenschaften, d.h. Feinstruktur und nominelle Molekulartrennschnittgrenze immer den gleichen Wert an.

Es gilt bei Membranen mit nomineller Trennschnittgrenzen von 10000 Daltons:

$$\frac{E_{375nm}}{E_{450nm}}=2,40—3,45$$

4.) Chromophor-Gruppentest
Meßprinzip

Durch Alkalisierung einer wässrig-Sauren Lösung bilden sich zusätzliche lichtabsorbierende chromophore Gruppen, die zu einer meßbaren Steigerung der Lichtabsorption führen.

Methode

Eine Wirkstofflösung wird soweit mit destilliertem Wasser verdünnt, daß sie in einer 1 cm Küvette bei 485 nm und fast voller Blende genau 100% Durchlässigkeit zeigt. Der pH-Wert dieser Lösung liegt zwischen 4 und 6.

Unter Kontrolle mit der Glaselektrode gibt man tropfenweise 1 n Kalilauge zu (ca. 1 Tropfen pro ml), bis der pH-Wert 9—10 beträgt. Die Lösung wird intensiv gemischt. Nach 20 Minuten wird erneut die Transparenz gemessen. Sie soll 19% betragen.

5.) Beeinflussung des Glutathionstatus der Leber im Rattenversuch (Anabole Wirksamkeit)

Männliche Wistar-Ratten des SPF-Stammes Han/Bö (120 g Körpergewicht) wurden um 9 Uhr vormittags mit 0,4 ml Equi-Thesin (Jensen Salsbury Laboratories, Kansas City, USA) pro 100 g Körpergewicht in Allgemeinnarkose versetzt, eröffnet und 0,3 $\mu$g des erfindungsgemäßen Wirkstoffes in 0,1 ml physiologischer Kochsalzlösung direkt in die vena protae injiziert. Nach 1, 2, 10 und 30 Minuten wurde der lobus dexter der Leber durch Frierstopp (1) entnommen und bis zur Analyse bei —150°C aufbewahrt. Die Bestimmung des Glutathionstatus erfolgte nach der unter (2) und (3) beschriebenen Methode. Reduziertes (GSH), oxidiertes (GSSG) und Gesamtglutathion (TG) wurden separat erfaßt und die gemischten Glutathiondisulfide (XSSG) nach der Formel TG-(GSH+2GSSG) errechnet. Die Ergebnisse sind in der folgenden Tabelle zusammengestellt.

4

(1) HOHORST, H. J., KREUTZ, F. H. und Bücher, Th. (1959); Biochem. Z. *332*, 18—46;
(2) HARISCH, G. und SCHOLE, J. (1971); Strahlentherapie *142*, 494—501;
(3) HARISCH, G. und SCHOLE, J. (1974); Z. Naturforsch. *29c*, 261—266;

TABELLE

Glutathionkonzentration der Rattenleber (Wistar, männlich, 120 g Körpergewicht) nach Verabreichung von 0,3 $\mu$g Wirkstoff direkt in die vena portae

Glutathionkonzentration Minuten nach Wirkstoffapplikation

| | 0 | 1 | 2 | 10 | 30 |
|---|---|---|---|---|---|
| TG | 8,02±0,66 $\mu$g/g Leber (7)* | 8,00±0,54 $\mu$g/g Leber (8) | — | 8,24±0,33 $\mu$g/g Leber (7) | 8,21±0,71 $\mu$g/g Leber (5) |
| GSH | 5,24±0,12 $\mu$g/g Leber (12) | 6,77±0,21** $\mu$g/g Leber (9) | 6,20±0,50** $\mu$g/g Leber (13) | 5,76±0,24** $\mu$g/g Leber (6) | 5,34±0,11 $\mu$g/g Leber (3) |
| GSSG | 0,161±0,02 $\mu$g/g Leber (10) | 0,116±0,01** $\mu$g/g Leber (10) | — | 0,120±0,04** $\mu$g/g Leber (12) | 0,138±0,01 $\mu$g/g Leber (3) |
| XSSG (als SH-Glutathion) | 2,46 $\mu$g/g Leber | 1,00 $\mu$g/g Leber | — | 2,24 $\mu$g/g Leber | 2.59 $\mu$g/g Leber |
| $\dfrac{\text{GSH}}{\text{GSSG}}$ | 32,5 | 58,4 | | 48,0 | 38,7 |

*=Tierzahl.
**=$p < 0,001$.
TG=Gesamtglutathion.
GSH=reduziertes Glutathion.
GSSG=oxidiertes Glutathion.
XSSG=gemischte Glutathiondisulfid.

Schon 1 Minute nach der Wirkstoffgabe tritt eine für die Einwirkung einer anabolen (synthesefördernden) Substanz charakteristische, hochsignifikante Zunahme des reduzierten Glutathions ein, unter gleichzeitiger Abnahme der oxidierten Verbindung und der gemischten Glutathiondisulfide. Gesamtglutathion bleibt unverändert. Nach einer unter genau gleichen Bedingungen vorgenommenen Insulininjektion (0,1 E) ist 10 Minuten nach der Hormongabe eine entsprechende Reduktion des Glutathionsystems zu beobachten. Eine Natriumchlorid-Injektion (0,1 ml einer physiologischen Kochsalz-Lösung) führt zu keiner signifikanten Änderung des Glutathionstatus.

6.) Schwimm-Leistungstest

Unter streng kontrollierten Prüfbedingungen ist dieser Test besonders geeignet zur Gütekontrolle der erfindungsgemäßen Wirksubstanz, da mit Hilfe dieses Tests sowohol katabole wie anabole Effekte erfaßt werden.

Versuchsanstellung

Für alle Versuche wurden weibliche Albino-Mäuse (NMRJ; 41—50 g Körpergewicht; Alter: 10 Monate) verwendet. Diese Tiere wurden etwa 10 Tage an die neuen Umgebungsbedingungen adaptiert und während dieser Zeit in Versuchsgruppen zu je 10 Tieren vergleichbaren Gewichts pro Käfig gehalten und Futter und Wasser ad libitum angeboten. Die Raumtemperatur lag bei 24°C, die relative Luftfeuchtigkeit bei 50% und die tägliche Beleuchtungsdauer betrug 12 Stunden. 16 Stunden vor dem Schwimmversuch wurde den Tieren das Futter entzogen.

Für den Schwimmversuch wurden um 9,30 Uhr morgens jeweils 10 Tiere zeitlich versetzt in ein Wasserbecken von 0,64 qm Oberfläche (Inhalt 0,32 m³) gebracht. Die Wassertemperatur betrug 20°C±1°C. Außerdem wurden dem Wasser 20 ml eines neutralen Detergens zugesetzt, um eine Luftpolsterbildung im Fell bei den schwimmenden Tieren zu verhindern. Die Schwimmzeit bis zur Erschöpfung (Untertauchen des Kopfes) wurde für jede Maus getrennt registriert. Anschließend wurden die Tiere mit einem Föhn getrocknet. Jede Gruppe kam nur einmal in den Versuch. Todesfälle traten bei diesem Versuch nicht auf. Die Wirkstoffverabreichung erfolgt in 0,2 ml einer physiologischen Kochsalzlösung pro 20 g Maus. Die damit verbundene Streßsituation wurde durch entsprechende Kontrollgruppen, die nur das Lösungsmittel erhielten, berücksichtigt.

Ergebnis

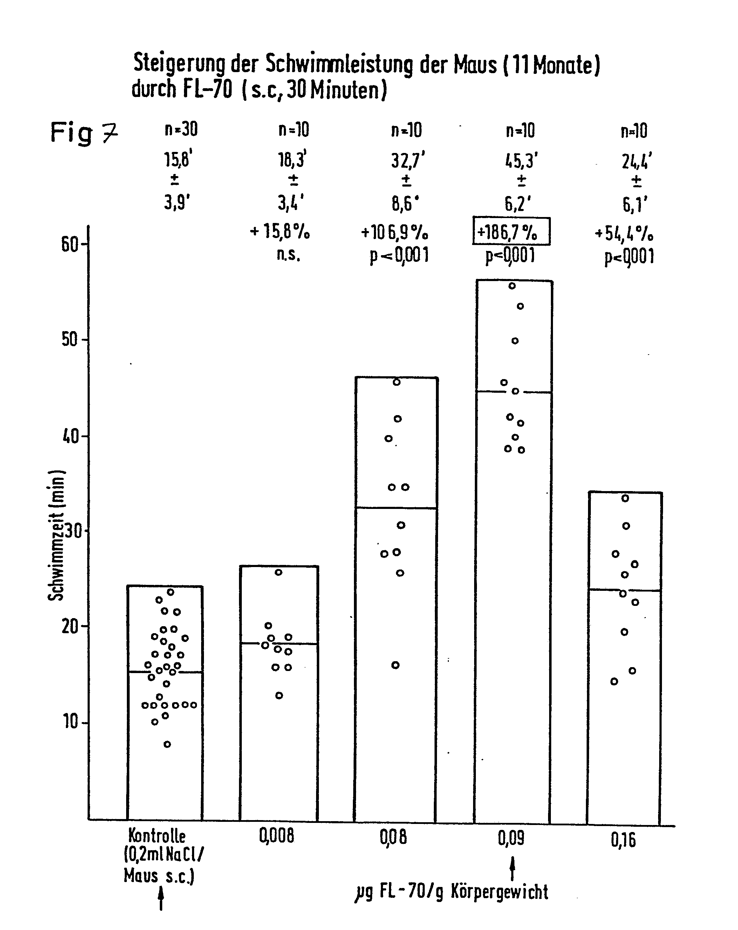

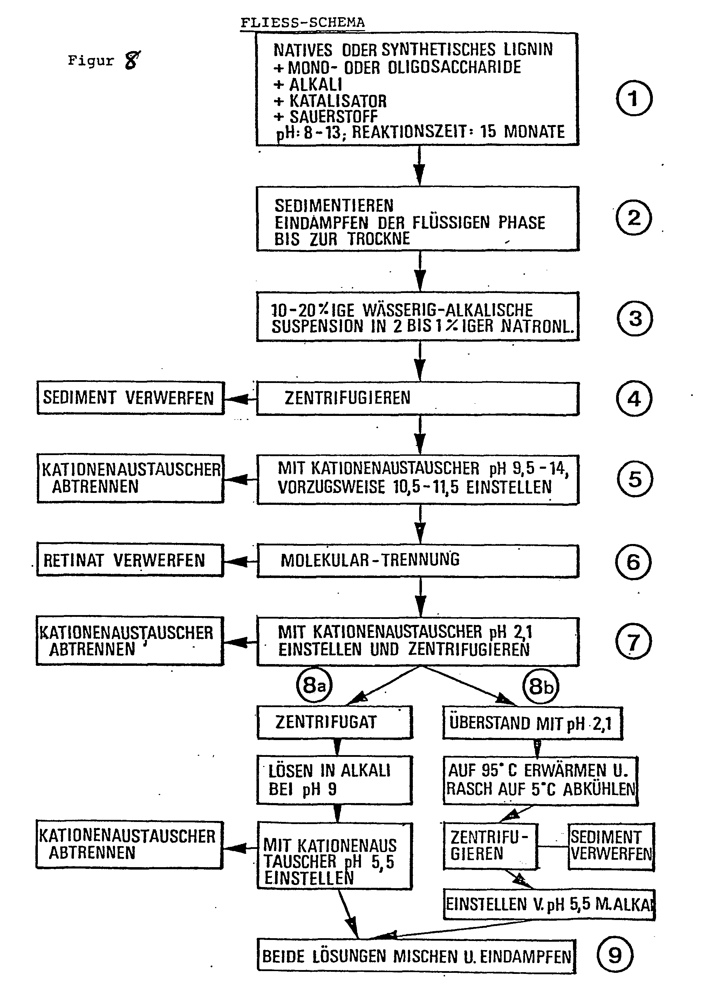

Durch 0,09 $\mu$g/g Tier wird die Schwimmleistung unter den gegebenen Prüfbedingungen um 186,7% gesteigert (vgl. Fig. 7).

Nachfolgend wird die Herstellung der erfindungsgemäßen Substanz anhand eines Beispiels erläutert.

Beispiel

20 kg pulverisiertes NORD's-Lignin und 2 kg einer pulverförmigen Mischung aus $Fe_2O_3$, Braunstein und Montmorillonit im Verhältnis 85:10:5 werden mit 2 n-Natrolauge und dann mit 0,3 n-Ammoniumhydroxidlösung zu einer rührfähigen Dispersion verdünnt. Man rührt 185 g d-Glucose ein und leitet dann in die bei Raumtemperatur gerührte Suspension durch eine Tauchfritte Sauerstoff ein. Nach ca. 15 bis 18 Monaten ist die Reaktion beendet. Der pH-Wert der Suspension ist dann um 1—2 Einheiten gestiegen. Man filtriert die ungelösten Anteile ab und dampft das Filtrat am Rotationsverdampfer unter Vakuum ein.

Ein Teil des getrockneten Filtrats wird in 10 Teilen 2 %iger Natronlauge suspendiert und dabei teilweise aufgelöst. Der ungelöste Anteil wird auf übliche Weise abfiltriert und verworfen. Durch Einrühren eines Kationenaustauschers in das wässrige Filtrat wird ein pH-Wert von 11,0 eingestellt. Der Kationenaustauscher wird anschließend abfiltriert.

Die wässrige Lösung wird unter einem Druck von 2,8 bar durch eine asymmetrische Druckfiltrationsmembran mit einer nominellen Molekulartrennschnittsgrenze von 20000 Daltons filtriert. Während der Filtration läßt man Schwingungen von 100 Hertz senkrecht zur Membranoberfläche einwirken.

In das erhaltene Filtrat wird ein Kationenaustauscher eingerührt, bis ein pH-Wert von 2,1 eingestellt ist. Man filtriert rasch vom Ionenaustauscher ab und zentrifugiert. Das gelartige Sediment und der klare Überstand werden durch Dekantieren getrennt.

a) Das gelartige Sediment wird in verdünnter Kalilauge bei pH 9 gelöst. Die klare Lösung wird mit einem Kationenaustauscher versetzt, nis ein pH-Wert von 5,5 erreicht ist.

b) Die überstehende Lösung von pH 2,1 wird mit einem Magnetron dielektrisch auf +95°C erwärmt und dann rasch auf +5°C abgekühlt. Das entstehende Sediment wird abzentrifugiert und verworfen. Durch Zugabe von Kaliumhydroxidlösung zum klaren Überstand wird unter Kontrolle mit der Glaselektrode pH 5,5 eingestellt.

Die nach a) und b) erhaltenen Lösungen werden vereinigt und lyophilisiert. Es verbleibt ein dunkelbrauner Feststoff.

Galenische Zubereitungen

Ampulle, Lösung, Spray, Sirup, Suppositorium, Stylus, Granulat, Tablette, Dragee, Kapsel, Salbe (Haft-Gel, -Creme, -Paste).

Anwendungsformen

i.m., i.v., s.c., i.a., lokal, oral, rektal, vaginal.

Ampullen

Konzentrationsbereich: 4 bis 4000 $\mu$g, bevorzugt 8 bis 40 $\mu$g

Lösungsmittel: 0,9 %ige NaCl-Lösung

Volumen: 1,0 ml

i.m., i.v., i.c., s.c., i.a.

Lokale Anwendung

Als Gel Creme, Spray, Haftpaste, Tropfen, Granulat u.ä.

Konzentration

0,05—10%

Standardwerte: 0,4—2%.

Orale Anwendung

Syrup, Lutschtablette, Pastille usw.

Konzentrationsbereich

0,5—3%.

# 0 035 586

Indikationsgebiete
  Entzündliche und degenerative sowie schmerzhafte Erkrankung an

— Haut und Schleimhat
  wie Verbrennungen, Dekubitus,
  Allergien, Pruritus,
  Stomatitis, Gingivitis, Parodontitis, Rhinitis,
  Konjunktivitis, Vaginitis,
  Ulzerationen im Gastrointestinaltrakt
— Gefäßen
  wie Venopathien, Ulcus cruris, hämorrhoidales
  Syndrom, Arteriosklerose
— Bindegewebe und Stützgewebe
  wie Rheuma, Arthrosen, Arthritiden, Osteoporose,
  Osteosynthese-Störungen
— Organen
  wie Leber, Herz, Gehirn, Skelettmuskel
  sowie

zur Prophylaxe und Therapie bei Regulationsstörungen des intermediären Stoffwechsels wie Prä-Diabetes, Diabetes, Proteinstoffwechsel-Störungen, mangelhafter Energiverwertung, verminderter Gewebereagibilität, verzögerter Wundheilung, verminderter Resistenz gegen Erreger und Tumoren.

Vergleich der relativen Wirkungen an Entzündungsmodell zwischen der erfindungsgemäßen Substanz (FL 70) und Phenylbutazon

| Modell | Dosierung | FL 70 | Phenylbutazon |
|---|---|---|---|
| Bolus-alba-Ödem | i.m. 3×10 mg/kg | 122,2 | 100 |
| Carageenin-Ödem | i.m. 3×10 mg/kg | 109 | 100 |
| Formalin-Ödem | i.m. 3×10 mg/kg | 241 | 100 |
| Dextran-Ödem | i.m. 3×10 mg/kg | 475 | 100 |
| Exsudationshemmung im Polyurethanschaumtest | i.p. 40 mg/kg | 266 | 100 |
| Chloroform-Reiztest | i.v., i.p. 40 mg/kg | 166 | 100 |
| Erythemverzögernde Wirksamkeit *epikutan* | e.c. 300 $\mu$g/cm² | 102 | 100 |

Akute Toxizität von FL 70 ($LD_{50}$ in mg/kg)

| Tierart | Applikation | $LD_{50}$ mg/kg |
|---|---|---|
| Maus | i.v. | 367,5 |
| | i.p. | 628 |
| | s.c. | 1080 |
| | p.o. | 12700 |
| Ratte | i.p. | 480 |
| | i.m. | 710 |
| | s.c. | 900 |
| | p.o. | 10200 |
| Hund | p.o. | >4000 |

7

**0 035 586**

Akute Toxizität von Phenylbutazon ($LD_{50}$ in mg/kg)

| Maus | i.v. | 123 |
| | i.p. | 336 |
| | p.o. | 680 |
| Ratte | i.p. | 215 |
| | p.o. | 467 |

Vergleich der akuten Toxizität von FL 70 und Phenylbutazon/PB

| Tierart | Applikation | Quotient $\left(\dfrac{\text{FL 70}}{\text{Phenylbutazon}}\right)$ |
|---|---|---|
| Maus | i.v. | ~4,2 |
| | i.p. | ~1,4 |
| | p.o. | >5,9 |
| Ratte | i.p. | ~2,7 |
| | p.o. | >8,5 |

Je nach Tierart und Applikationsart ist Phenylbutazon ~1,4 bis >8,5 mal toxischer als FL 70.

**Patentansprüche**

1. Wasserlöslicher, niedermolekularer, lignoider Wirkstoff, dadurch gekennzeichnet, daß er

a) als Feststoff ein ESR-Spektrum gemäß Abb. 1,
b) in wässrig-neutraler Lösung ein ESR-Spektrum gemäß Abb. 2, in wässrig-alkalischer Lösung bei pH 10 ein ESR-Spektrum gemäß Abb. 3,
c) in wässriger Lösung ein Fluoreszenzspektrum gemäß Abb. 4 bis 6,
d) beim Chromophor-Gruppentest bei Erhöhung des pH-Wertes vom sauren in den basischen Bereich einen Rückgang der Transparenz von 100% auf etwa 20% und
e) beim Glutathion-Redoxstatustest nach Hohorst et al, 1959, Biochem. Z. 332, 18—46) ab 60 sec. nach der Applikation eine charakteristische Zunahme des reduzierten Glutathions bei gleichzeitiger Abnahme des oxidierten Glutathions und der gemischten Glutathiondisulfide gemäß Tabelle (vgl. S 12) zeigt.

2. Verfahren zur Herstellung des Wirkstoffs nach Anspruch 1, dadurch gekennzeichnet, daß man

a) künstliche oder native Monolignole in wässriger oder wässrig/organischer, saurer oder neutraler Lösung unter Sauerstoffzufuhr und unter Zusatz von wasserlöslichen Polysacchariden einer enzymatischen Dehydrierungspolymerisation unterwirft,
b) die anfallenden Dehydrierungspolymerisate (DHPs) in alkalischem Milieu in Gegenwart von Sauerstoff und in Anwesenheit eines Oxidationskatalysators bis zur Bildung wasserlöslicher, gefärbter Umsetzungsprodukte oxidativ depolymerisiert und gleichzeitig repolymerisiert,
c) von dem aus wasserunlöslichen, oxidativ noch nicht umgesetzten DHPs und wasser un löslichen, umgesetzten Reaktionsprodukten bestehenden Gemisch den wasserlöslichen Anteil abtrennt und verwirft,
d) den Überstand klar filtriert und unter Einwirkung mechanischer Schwingungen einer Molekularfiltration an Ultrafiltrationsmembranen mit nomineller Trennschnittgrenzen von 5000 bis 35000 Daltons unterwirft,
e) das alkalische Molekularfiltrat auf einen pH-Wert von 4,5—6,0 einstellt und das Filtrat gewinnt oder
f) den pH-Wert auf etwa 2,1 bis 3,4 einstellt, anschließend zentrifugiert, die bei der Zentrifugation als Gel erhaltene säureempfindliche Fraktion abgrenzt, mittels Alkali auf etwa pH 9 einstellt und die dabei entstehende klare wässrige Lösung zur Entfernung von überschüssigem Alkali mittels eines Kationenaustauschers auf pH 3,5 bis 6,9 zurückstellt (Fraktion 1), die als Überstand zer Zentrifugation erhaltene säurestabile Fraktion einem Thermoschock aussetzt und die erhaltene leicht getrübte Lösung vom unlöslichen Niederschlag abtrennt, den klaren Überstand mittels Alkali auf pH 3,5 bis 6,9 einstellt und diese Fraktion 2 mit der Fraktion 1 vereinigt.

3. Verfahren nach Anspruch 2, dadurch gekennzeichnet, daß die Verfahrensstufe a) in Gegenwart von 1,5 bis 9,5 Gew.-% wasserlöslicher Polysaccharide durchgeführt wird.
4. Verfahren nach einem Ansprüche 2 bis 3, dadurch gekennzeichnet, daß man die Verfahrensstufe a) mittels Reduktase oder Peroxydase durchführt.

8

5. Verfahren nach einem der Ansprüche 2 bis 4, dadurch gekennzeichnet, daß man die Verfahrensstufe b) unter Einleiten und/oder katalytischer Erzeugung von Sauerstoff durchführt.

6. Verfahren nach einem der Ansprüche 2 bis 5, dadurch gekennzeichnet, daß die Verfahrensstufe b) in Gegenwart von $MnO_2$, $\alpha$-$Fe_2O_3$ und/oder hochdispersem $SiO_2$ als Oxidationskatalysator durchgeführt wird.

7. Verfahren nach einem der Ansprüche 2 bis 6, dadurch gekennzeichnet, daß die Verfahrensstufe b) in einem Temperaturbereich von +10 bis +60°C durchgeführt wird.

8. Verfahren nach einem der Ansprüche 2 bis 7, dadurch gekennzeichnet, daß die Verfahrensstufe b) bis zur Bildung von 25 bis 95% wasserlöslicher Umsetzungsprodukte, bezogen auf die eingesetzten DHPs, durchgeführt wird.

9. Verfahren nach einem der Ansprüche 2 bis 8, dadurch gekennzeichnet, daß man in der Verfahrensstufe d) im Schallbereich die Schwingungen vorzugsweise senkrecht und im Ultraschallbereich vorzugsweise parallel zur Molekularfiltrationsmembran einwirken läßt.

10. Verfahren nach einem der Ansprüche 2 bis 9, dadurch gekennzeichnet, daß man in der Verfahrensstufe d) eine alkali-beständige Molkularfiltrationsmembran mit nominellen Trennschnittgrenzen zwischen 10 000 bis 25 000 Dalton verwendet.

11. Verfahren nach einem der Ansprüche 2 bis 10, dadurch gekennzeichnet, daß man in der Verfahrensstufe f) die säurestabile Fraktion auf +95°C erhitzt und anschließend rasch auf +5°C abkühlt.

12. Verfahren nach einem der Ansprüche 2 und 5 bis 11, dadurch gekennzeichnet, daß man anstelle von synthetischen Dehydrierungspolymerisaten in der Verfahrensstufe b) native Dehydrierungspolymerisate als Ausgangsmaterial einsetzt.

13. Pharmazeutische Zubereitung, enthaltend die Wirksubstanz nach einem der Ansprüche 1 bis 12, neben üblichen Zusatz- und/oder Trägerstoffen.

14. Pharmazeutische Zubereitung zur lokalen Anwendung enthaltend die Wirksubstanz nach einem der Ansprüche 1—12 zusammen mit Salicylsäurederivaten, Aminosäuren oder Vitaminen neben üblichen Zusatz- und/oder Trägerstoffen.

**Revendications**

1. Substance active lignoïde à faible poids moléculaire et soluble dans l'eau, caractérisée en ce qu'elle présente:

a) sous forme de matière solide un spectre RSE selon la figure 1,

b) en solution aqueuse neutre un spectre RSE selon la figure 2, en solution aqueuse alcaline de pH 10 un spectre RSE selon la figure 3,

c) en solution aqueuse un spectre de fluorescence slon les figures 4 à 7,

d) lors de l'essai de groupements chromophores, par élévation de la valeur du pH du domaine acide au domaine basique, une diminution de transparence de 100% à environ 20%, et

e) dans l'essai de l'état redox du glutathion selon Hohorst et al., (1959, Biochem. Z. 332, 18—46) à partir de 60 secondes après l'application une augmentation caractéristique du glutathion réduit avec diminution simultanée du glutathion oxydé et du disulfure de glutathion mélangé selon le Tableau (voir page 10).

2. Procédé de préparation de la substance active selon la revendication 1, caractérisé en ce que:

a) on soumet des monolignols d'origine synthétique ou naturelle, dans une solution aqueuse ou aqueuse/organique, acide ou neutre, sous courant d'oyxgène et avec adjonction de polysaccharides solubles dans l'eau, à une polymérisation déshydratante enzymatique,

b) on dépolymérise par oxydation et on repolymérise simultanément, en milieu alcalin en présence d'oxygène et en présence d'un catalyseur d'oxydation, les polymérisats de déshydratation (PDHs) précipités obtenus, jusqu'à formation d'un produit de réaction coloré et soluble dans l'eau,

c) on sépare et on élimine la partie soluble dans l'eau du mélange constitué par les PDHs insolubles dans l'eau non encore transformés par oxydation et les produits insolubles dans l'eau ayant réagi,

d) on filtre le surnageant clair et on le soumet sous vibrations mécaniques à une filtration moléculaire par des membranes d'ultrafiltration à limites de séparation nominales de 5.000 à 35.000 daltons,

e) on ajuste le filtrat moléculaire alcalin à un pH 4,5—6,0, et on récupère le filtrat, ou bien

f) on règle la valeur du pH de 2,1 à 3,4, puis on sépare par centrifugation la fraction sensible à l'acide obtenue sous forme de gel, on règle le pH à environ 9 au moyen d'alcali et on ramène de la solution aqueuse claire obtenue (fraction 1) le pH à une valeur de 3,5 à 6,9, pour éliminer l'alcali en excès, au moyen d'un échangeur de cations, on soumet le surnageant de la fraction stable aux acides obtenue par centrifugation à un choc thermique et on sépare la solution l'égèrement trouble obtenue du résidu insoluble, on règle le pH du surnageant clair au moyen d'alcali à une valeur de 3,5 à 6,9 et on purifie cette fraction 2 avec la fraction 1.

3. Procédé selon la revendication 2, caractérisé en ce que l'étape a) du procédé est réalisée en présence de 1,5 à 9,5% en poids de polysaccharide soluble dans l'eau.

4. Procédé selon l'une des revendications 2 à 3, caractérisé en ce que l'étape a) du procédé est réalisée au moyen de réductase ou de peroxydase.

5. Procédé selon l'une des revendications 2 à 4, caractérisé en ce que l'étape b) du procédé est réalisée en introduisant et/ou en produisant catalytiquement de l'oxygène.

6. Procédé selon l'une des revendications 2 à 5, caractérisé en ce que l'étape b) du procédé est réalisée en présence de $MnO_2$, $\alpha$-$Fe_2O_3$ et/ou de $SiO_2$ hautement dispersé en tant que catalyseur d'oxydation.

7. Procédé selon l'une des revendications 2 à 6, caractérisé en ce que l'étape b) du procédé est réalisée à une température de +10 à +60°C.

8. Procédé selon l'une des revendications 2 à 7, caractérisé en ce que l'étape b) du procédé est réalisée jusqu'à ce que l'on obtienne de 25 à 95% d'un produit de réaction soluble dans l'eau, par rapport au PDHs utilisés.

9. Procédé selon l'une des revendications 2 à 8, caractérisé en ce qu'au cours de l'étape d) du procédé, on laisse les vibrations agir de préférence perpendiculairement dans la zone sonore et de préférence parallèlement dans la zone des ultrasons, par rapport à la membrane de filtration moléculaire.

10. Procédé selon l'une des revendications 2 à 9, caractérisé en ce qu'on utilise à l'étape d) du procédé une membrane de filtration moléculaire résistant aux alcalis et dont les limites de séparation nominales sont de 10.000 à 25.000 daltons.

11. Procédé selon l'une des revendications 2 à 10, caractérisé en ce qu'à l'étape f) du procédé, on chauffe à +95°C la fraction stable aux acides et en ce qu'on la refroidit ensuite rapidement à +5°C.

12. Procédé selon l'une des revendications 2 et 5 à 11, caractérisé en ce que l'on utilise à l'étape b) du procédé et à la place de polymérisats de déshydratation synthétiques des polymérisats de déshydratation d'origine naturelle en tant que matériaux de départ.

13. Préparation pharmaceutique contenant la substance active selon l'une des revendications 1 à 12, avec des additifs et/ou véhicules habituels.

14. Préparation pharmaceutique pour application locale, comprenant la substance active selon l'une des revendications 1 à 12, en combinaison avec des dérivés d'acide salicyclique, des acides aminés ou des vitamines, et avec des additifs et véhicules usuels.

## Claims

1. Water-soluble, low molecular lignoid active agent, characterized in that it shows

a) as solid an ESR spectrum according to Fig. 1,
b) in aqueously neutral solution an ESR spectrum according to Fig. 2, in aqueously alkaline solution at pH 10 an ESR spectrum according to Fig. 3,
c) in aqueous solution a fluorescence spectrum according to Fig. 4 to 6,
d) in the chromophoric group test with increase of the pH value from the acid to the basic range a decline of transparency from 100% to about 20% and
e) in the glutathione redox status test according to Hohorst et al., 1959, Biochem. Z. 332, 18—46, beginning with 60 sec. after the application a characteristic increase of the reduced glutathione with simultaneous decline of the oxidized glutathione and the mixed glutathionedisulfides according to table (cf. page 12).

2. Process for the preparation of the active agent according to claim 1, characterized in that

a) artificial or native monolignols in aqueous or aqueous/organic, acid or neutral solution with oxygen supply and with addition of water-soluble polysaccharides are subjected to an enzymatic dehydration polymerization,
b) the resulting dehydration polymers (DHPs) are oxidatively depolymerized and simultaneously repolymerized in an alkaline medium in the presence of oxygen and in the presence of an oxidation catalyst until formation of water-soluble colored reaction products,
c) the water-soluble portion is separated and rejected from the mixture consisting of water-insoluble, oxidatively not yet reacted DHPs and water-insoluble reacted reaction products.
d) the supernatant is filtered to clarity and is subjected under the influence of mechanical vibrations to a molecular filtration on ultra-filtration membranes having nominal separating-cut limits of 5000 to 35,000 Daltons,
e) the alkaline molecular filtrate is adjusted to a pH value of 4.5—6.0 and the filtrate is recovered or
f) the pH value is adjusted to about 2.1 to 3.4, it is subsequently centrifuged, the acid-sensitive fraction which is obtained from the centrifugation as a gel is separated, is adjusted to a pH value of about 9 by means of alkaline and the clear aqueous solution which is obtained thereby is readjusted in order to remove excess alkaline by means of a cation exchanger to a pH value of 3.5 to 6.9 (fraction 1), the acid-stable fraction which is obtained as supernatant of the centrifugation is subjected to a thermo shock and the obtained, slightly cloudy solution is separated from the insoluble precipitate, the clear supernatant is adjusted by means of alkaline to a pH value of 3.5 to 6.9 and this fraction 2 is combined with fraction 1.

3. Process according to claim 2, characterized in that the process step a) is performed in the presence of 1.5 to 9.5% by weight of water-soluble polysaccharides.

4. Process according to one of claims 2 to 3, characterized in that the process step a) is performed by means of reductase or peroxydase.

5. Process according to one of claims 2 to 4, characterized in that the process step b) is performed under supply and/or catalytic generation of oxygen.

6. Process according to one of claims 2 to 5, characterized in that the process step b) is performed in the presence of $MnO_2$, $\alpha$-$Fe_2O_3$ and/or highly dispersed $SiO_2$ as oxidation catalyst.

7. Process according to one of claims 2 to 6, characterized in that the process step b) is performed in a temperature range of from +10 to +60°C.

8. Process according to one of claims 2 to 7, characterized in that the process step b) is performed until the formation of 25 to 95% of water-soluble reaction products, based on the supplied DHPs.

9. Process according to one of claims 2 to 8, characterized in that in the process step d) the vibrations are allowed to act on in the sound range preferably vertically and in the ultra sound range preferably parallel to the molecular filtration membranes.

10. Process according to one of claims 2 to 9, characterized in that in the process step d) an alkaline-resistant molecular filtration membrane having nominal separating-cut limits between 10,000 and 25,000 Daltons is used.

11. Process according to one of claims 2 to 10, characterized in that in the process step f) the acid-stable fraction is heated to +95°C and is then quickly cooled to +5°C.

12. Process according to one of claims 2 and 5 to 11, characterized in that instead of synthetic dehydration polymers in the process step b) native dehydration polymers are used as starting material.

13. Pharmaceutical preparation containing the active substance according to one of the claims 1 to 12 together with the usual additive and/or carrier substances.

14. Pharmaceutical preparation for local application, containing the active substance according to one of claims 1 to 12 together with salicylic acid derivatives, amino acids or vitamins besides the usual additive and/or carrier substances.

FIG.1

# FIG. 2

# FIG.3

# Emissionsspektrum
# Charge I
# Figur 4

Lösungsmittel: . Wasser

Meßverdünnung: 0,00097 %

Schichtdicke: 10 mm

Gerät: PE 3000

Spalt Ex=5mm; Spalt Em=5mm

Scan: 30 nm/min

Exp.: x 0

TC: 6 sec.

Papier: 10mm/min, 5mV ≙ 100%

$\lambda$ Ex = 280 nm

$\lambda$ Em = 300 - 550 nm

$\lambda$ max. = 450 nm

# Emissionsspektrum
# Charge II
# Figur 5

| | |
|---|---|
| Lösungsmittel: | Wasser |
| Meßverdünnung: | 0,00097% |
| Schichtdicke: | 10 mm |
| Gerät: | PE 3000 |
| Spalt Ex=5mm; | Spalt Em =5mm |
| Scan: | 30 nm/min |
| Exp.: | x 0 |
| TC : | 6 sec. |
| Papier: 10mm/min, 5mV ≙ 100% | |
| $\lambda$ Ex = | 280 nm |
| $\lambda$ Em = | 300 - 550 nm |
| $\lambda$ max. = | 450 nm |

# Emissionsspektrum
## Charge III
## Figur 6

Lösungsmittel:    Wasser
Meßverdünnung:    0,00097%
Schichtdicke :    10 mm
Gerät :    PE 3000
Spalt Ex=5mm; Spalt Em=5mm
Scan:    30 nm/min
Exp.:    x0
TC :    6 sec.
Papier: 10 mm/min, 5 mV ≙ 100%
$\lambda$ Ex  =    280 nm
$\lambda$ Em  =    300 – 550 nm
$\lambda$ max =    450 nm

Steigerung der Schwimmleistung der Maus (11 Monate)
durch FL-70 (s.c, 30 Minuten)

Fig 7

**0 035 586**

Figur 8

**NATIVES ODER SYNTHETISCHES LIGNIN
+ MONO- ODER OLIGOSACCHARIDE
+ ALKALI
+ KATALISATOR
+ SAUERSTOFF
pH: 8-13; REAKTIONSZEIT: 15 MONATE** ①

**SEDIMENTIEREN
EINDAMPFEN DER FLÜSSIGEN PHASE
BIS ZUR TROCKNE** ②

**10-20%IGE WÄSSERIG-ALKALISCHE
SUSPENSION IN 2 BIS 1%IGER NATRONL.** ③

**SEDIMENT VERWERFEN** ← **ZENTRIFUGIEREN** ④

**KATIONENAUSTAUSCHER
ABTRENNEN** ← **MIT KATIONENAUSTAUSCHER pH 9,5-14,
VORZUGSWEISE 10,5-11,5 EINSTELLEN** ⑤

**RETINAT VERWERFEN** ← **MOLEKULAR-TRENNUNG** ⑥

**KATIONENAUSTAUSCHER
ABTRENNEN** ← **MIT KATIONENAUSTAUSCHER pH 2,1
EINSTELLEN UND ZENTRIFUGIEREN** ⑦

⑧a     ⑧b

**ZENTRIFUGAT**     **ÜBERSTAND MIT pH 2,1**

**LÖSEN IN ALKALI
BEI pH 9**     **AUF 95°C ERWÄRMEN U.
RASCH AUF 5°C ABKÜHLEN**

**KATIONENAUSTAUSCHER
ABTRENNEN** ← **MIT KATIONENAUS
TAUSCHER pH 5,5
EINSTELLEN**    **ZENTRIFU-
GIEREN**   **SEDIMENT
VERWERFEN**

**EINSTELLEN V. pH 5,5 M.ALKA[**

**BEIDE LÖSUNGEN MISCHEN U. EINDAMPFEN** ⑨